# FASCICULE DE BREVET EUROPEEN

(11) **EP 2 967 962 B1**
(45) Date de publication et mention de la délivrance du brevet: **02.09.2020**
(21) Numéro de dépôt: 14718635.7
(22) Date de dépôt: 24.02.2014
(51) Int. Cl.: A61F 5/01, A61F 5/058

(54) **ORTHESE TALO-TARSIENNE**
TALOTARSALE ORTHESE
TALOTARSAL ORTHOSIS

(30) Priorité: 12.03.2013 FR 1352176
(43) Date de publication de la demande: 20.01.2016
(73) Titulaire: Cizeta Medicali France, 18200 St. Amand-Montrond (FR)
(72) Inventeur: DUBOURG, Charles, 18200 Meillant (FR)
(74) Mandataire: Gauchet, Fabien Roland
(86) Numéro de dépôt international: PCT/FR2014/050380
(87) Numéro de publication internationale: WO 2014/140440

(56) Documents cités:
- DE-A1- 19 722 118
- FR-A1- 2 766 360
- US-A- 4 862 900
- US-A1- 2004 236 259
- US-A1- 2012 238 928
- US-B1- 6 793 640

## Description

### DOMAINE TECHNIQUE DE L'INVENTION

L'invention se rapporte au domaine des orthèses rigides destinées à immobiliser la cheville et le pied, pour des applications médicales ou sportives, et/ou pour des personnes blessées au niveau de la cheville.

### ETAT DE LA TECHNIQUE ANTERIEURE

Les orthèses connues destinées à immobiliser la cheville et le pied sont généralement constituées de plusieurs coques rigides reliées les unes aux autres par des sangles ou bandeaux qui en outre permettent de les ajuster autour de la cheville et sous le talon. Les coques rigides sont amovibles et combinées à une sous-talonnière réglable et ajustable.

Il s'agit dans tous les cas de maintenir la cheville dans une position donnée, fixe, afin par exemple de stabiliser le ligament latéral externe à la cheville lorsqu'il est endommagé. Les pathologies concernées sont : les entorses qualifiées de bénignes à moyennes ; les blessures capsulo ligamentaires aiguës de la cheville ; les épanchements synoviaux ; l'instabilité chronique ou la laxité chronique.

Le document WO1994/005236 divulgue une orthèse adaptée à ce type de pathologies, qui comprend une zone supérieure disposée au-dessus de la cheville et une zone inférieure placée sous une partie du pied. Les zones comprennent des éléments rigides et sont reliées entre elles par des zones souples et/ou des attaches. Cependant cette attelle recouvre la zone où un oedème est développé (en cas d'entorse), ce qui pose un problème car cela induit une douleur chez le porteur de l'orthèse. De plus une telle orthèse est relativement complexe à fabriquer. En outre l'immobilisation des malléoles est réalisée à partir du bas de la jambe combinée à la partie proximale du pied.

Le document US 2002/0029009 décrit une coque relativement complexe puisque constituée d'une partie à placer sous le pied combinée à deux flancs latéraux reliés par des sangles. Encore une fois la zone touchée par un œdème est ici recouverte par l'un des flancs latéraux, ce qui pose problème. En outre et de façon classique, cet ensemble permet une immobilisation du bas de la jambe combinée à une immobilisation de la partie proximale du pied.

On connaît encore la demande GB 2 443 557 qui divulgue une coque rigide amovible dont la forme épouse celle de la partie postérieure de la cheville, combinée à au moins une autre partie rigide destinée à être placée sur le devant de la cheville. On réalise ainsi une sorte de carcan rigide qui enveloppe la cheville et une partie du pied. Ce type de réalisation n'est pas satisfaisant car il est en contact avec presque toute la surface à immobiliser et de ce fait il peut créer une douleur notamment sur la ou les oedèmes du patient. Par ailleurs une telle coque étant ajustée sur le patient, plusieurs tailles doivent être prévues afin d'épouser au mieux la forme de la cheville et du pied du patient.

Une orthèse selon le préambule de la revendication 1 est connue de FR 2 766 360. Cette attelle est constituée par une coque en matière plastique relativement rigide qui recouvre en partie la jambe et le pied. A la jonction de la partie jambe et de la partie pied de l'attelle sont ménagées deux ouvertures opposées en regard respectivement des deux malléoles de la cheville. En outre, la partie jambe de l'attelle comporte à l'arrière une ouverture oblongue qui permet de dégager le tendon d'Achille en s'étendant depuis la jonction tendino-musculaire du triceps sural à son insertion distale osseuse sur le calcanéum.

L'ensemble des orthèses connues sont basées sur l'immobilisation combinée du bas de la jambe et d'une partie du pied, ce qui n'est pas satisfaisant notamment parce qu'elles recouvrent et frottent le ou les oedemes développées sur le patient. De plus les orthèses connues doivent être portées avec une chaussure pour être totalement efficaces. La chaussure complète en fait l'immobilisation induite par l'attelle.

### EXPOSE DE L'INVENTION

L'invention vise à remédier aux inconvénients de l'état de la technique et notamment à proposer une nouvelle orthèse (ou attelle) qui peut être portée et efficace sans chaussure. Par ailleurs l'immobilisation prévue selon l'invention, est opérée au niveau du talon et non plus au niveau du bas de la cheville et du pied.

Pour ce faire est proposé selon l'invention une orthèse talo-tarsienne destinée à immobiliser l'articulation de la cheville, comprenant plusieurs zones rigides intérieurement recouvertes d'une gaine de contact avec au moins la cheville, au moins un bandeau de liaison destiné à fixer l'orthèse de façon amovible et réglable sur la cheville. Lesdites zones rigides sont destinées à immobiliser le calcanéum et le cuboïde. L'orthèse comprend deux zones de soutien rétro-malléolaire ce qui permet de dégager les deux malléoles.

On réalise ainsi une stabilisation du calcanéum et du cuboïde ; un tel blocage est efficace sur la cheville et le pied dans leur ensemble ; de plus un dégagement des deux malléoles est simultanément opéré.

De façon intéressante, le dégagement de la malléole latérale évite tout contact de l'orthèse avec des oedèmes formés à proximité.

Par ailleurs, l'orthèse selon l'invention comprend une première zone rigide destinée à soutenir le soléaire. Cette zone entoure donc l'arrière inférieur de la jambe tout en ménageant une ouverture au niveau du tendon d'Achille.

De façon plus précise, conformément à l'invention, ladite ouverture présente une forme en V s'ouvrant depuis l'arrière de la jambe vers le talon, de manière à ce que le talon soit dégagé, non recouvert par l'orthèse.

De façon intéressante, l'orthèse selon l'invention comprend une troisième zone rigide recouvrant partiellement la surface inférieure du pied, depuis le talon jusqu'au cuboïde. Il s'agit ici d'une zone destinée à soutenir le médio-pied.

Selon une autre caractéristique de l'invention, l'orthèse comprend au moins un bandeau de liaison pourvu de surfaces auto-agrippantes et prévu pour entourer circonférentiellement la jambe. Tout moyen techniquement équivalent peut être prévu sans sortir du cadre de l'invention. Il s'agit dans tous les cas d'assujettir et de fixer l'orthèse de façon amovible sur la zone concernée.

Conformément à une caractéristique additionnelle de l'invention, l'orthèse comprend au moins un bandeau de liaison pourvu de surfaces auto-agrippantes et prévu pour entourer la face dorsale du pied. Tout moyen techniquement équivalent peut être prévu sans sortir du cadre de l'invention.

En outre, ladite gaine de contact recouvre toute la surface intérieure de l'orthèse. Elle est destinée à rendre plus confortable le contact entre l'orthèse et la peau du patient.

En fonction des circonstances, ladite gaine peut présenter une épaisseur comprise entre environ 2 mm et environ 6 mm. L'homme de métier choisira l'épaisseur appropriée. La gaine peut être réalisée en une mousse de PE.

Avantageusement lesdites zones rigides sont constituées en PE ou en une autre matière plastique moulable et rigide. Il est donc particulièrement aisé de fabriquer une telle coque rigide.

Selon un mode préféré de réalisation de l'invention, lesdites zones rigides sont monobloc.

### BREVE DESCRIPTION DES FIGURES

D'autres caractéristiques, détails et avantages de l'invention ressortiront à la lecture de la description qui suit, en référence aux figures annexées, qui illustrent :
- la figure 1, une vue en perspective d'une orthèse vue de l'intérieur, selon un mode de réalisation de l'invention ;
- la figure 2, une vue en perspective d'une orthèse vue de l'extérieur, selon un mode de réalisation de l'invention ;
- la figure 3, une coupe longitudinale d'une orthèse selon un mode de réalisation de l'invention ; et
- la figure 4, une vue de l'arrière de l'orthèse.

Pour plus de clarté, les éléments identiques ou similaires sont repérés par des signes de référence identiques sur l'ensemble des figures.

### DESCRIPTION DETAILLEE D'UN MODE DE REALISATION

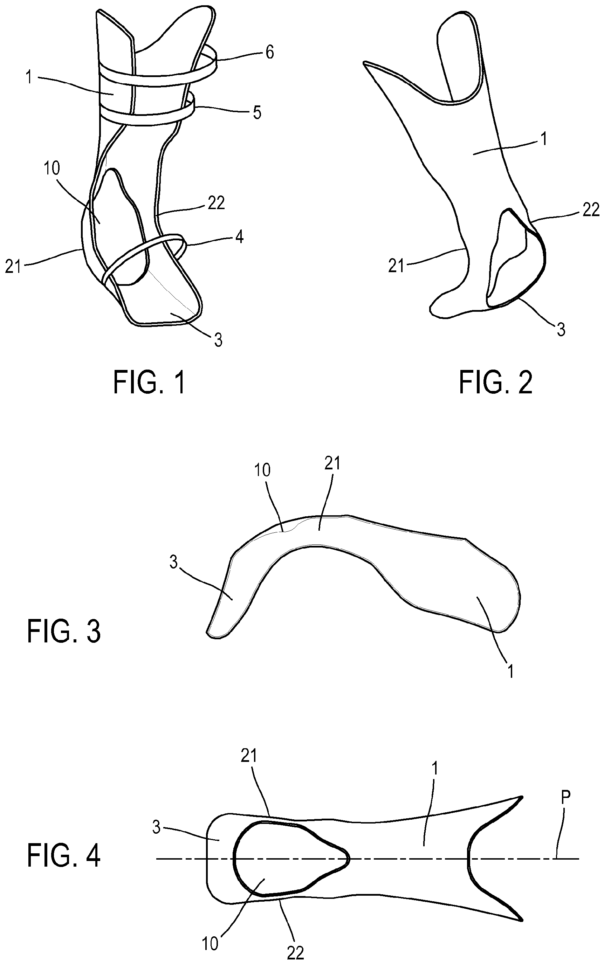

La figure 1 illustre une orthèse selon l'invention, de forme anatomique, destinée par exemple à immobiliser une cheville fragilisée par un choc ou une tension inappropriée. Cette orthèse présente une forme anatomique qui correspond donc partiellement à la surface extérieure du bas de la jambe et d'une partie du pied, proche du talon. L'orthèse comprend plusieurs zones rigides 1, 21, 22, 3. Elle est recouverte intérieurement d'une couche ou gaine par exemple en une mousse visco-élastique (non représentée ni référencée). Cette gaine est en contact direct avec la peau ou une chaussette ou un bas qui recouvre la cheville et/ou le pied du patient. La gaine est destinée à améliorer le confort du patient car l'orthèse est préférentiellement réalisée en un matériau plastique rigide tel que du polyéthylène. Comme il sera explicité plus loin, une telle gaine permet de diminuer les points de pression sur et autour de la cheville du patient. Elle recouvre préférentiellement toute la surface dite intérieure de l'orthèse. Par « surface intérieure » on entend la surface en vis-à-vis de et en contact avec la cheville à protéger.

Par ailleurs l'orthèse comprend au moins un et de préférence trois bandeaux de liaison 4, 5, 6 destinés à la fixer de façon amovible et réglable, sur et autour de la cheville.

**A** titre préféré, le bandeau de liaison est pourvu de surfaces auto agrippantes, de type Velcro ®, ce qui permet de l'ajuster facilement sur la cheville. Un tel bandeau peut être facilement mis en place et enlevé vis-à-vis de la cheville. Tout moyen techniquement équivalent entre bien entendu dans le cadre de l'invention.

**Un** premier bandeau de liaison 4 peut être prévu au niveau du pied, plus précisément avec la face dorsale du pied. Il coopère avec l'une des zones rigides, comme il sera explicité ci-après.

Un ou plusieurs autres bandeaux rigides 5, 6 peuvent coopérer avec une autre zone de l'orthèse, pour entourer le mollet selon sa circonférence.

Des moyens de liaison entre les bandeaux 4, 5, 6 et les zones rigides sont prévus, connus en eux-mêmes.

De façon particulièrement intéressante, l'orthèse anatomique selon l'invention comprend des zones rigides qui sont destinées à immobiliser le calcanéum et le cuboïde. Ceci est une façon nouvelle et inventive d'immobiliser une cheville. L'orthèse est sensiblement symétrique vis-à-vis d'un plan passant par le tibia et le milieu du talon.

Plus précisément, une première zone rigide 1 est prévue ; elle est destinée à soutenir l'arrière inférieur de la jambe, au niveau du mollet. Autrement dit, la première zone constitue un soutien rigide du soléaire. La première zone 1 présente avantageusement une forme sensiblement semi-cylindrique, avec préférentiellement deux avancées vers le genou afin de mieux entourer la zone concernée. En outre la première zone 1 présente une ouverture 10 au niveau du tendon d'Achille. De façon plus précise l'ouverture 10 est en forme de V qui s'ouvre vers le talon. Le talon est ainsi dégagé, non recouvert par l'orthèse, ce qui évite de le blesser.

L'orthèse selon l'invention comprend en outre deux zones 21, 22 de soutien rétro-malléolaire. Ces deux zones 21,22 sont donc disposées symétriquement vis-à-vis du plan de symétrie de l'orthèse, et elles sont chacune destinées à recouvrir une zone postérieure (c'est-à-dire à l'arrière) à la malléole, respectivement à la malléole médiane et à la malléole latérale. « A l'arrière » doit être compris comme dans le sens opposé « à l'avant » qui correspond aux phalanges du pied. Ces deux zones définissent par ailleurs les limites de l'ouverture 10 et elles constituent une liaison entre la première zone 1 et une troisième zone 3 de soutien du médio-pied.

Comme son nom l'indique la troisième zone 3 permet de soutenir le pied, par le dessous, sous le calcaneum et le cuboïde. Autrement dit la troisième zone recouvre partiellement la surface inférieure du pied, depuis le talon jusqu'au cuboïde.

L'homme de métier choisira les dimensions appropriées correspondant par exemple à deux ou trois morphologies moyennes : enfant, petit adulte, grand adulte. Les bandeaux 4, 5, 6 ainsi qu'éventuellement l'épaisseur de la gaine de contact, permettront d'adapter de façon précise l'orthèse à toutes les morphologies. A titre d'exemple la plus grande dimension de l'orthèse, ou hauteur, mesurée selon le plan P de symétrie, peut être comprise entre 20 cm et 30 cm. Ceci correspond à la distance entre le sol et la moitié du mollet.

La figure 3 est une coupe par le plan de symétrie P de l'orthèse, et elle montre bien les différentes zones rigides et leurs dispositions respectives.

La figure 4, vue arrière de l'orthèse, met en évidence sa symétrie vis-à-vis du plan P. L'ouverture 10 y est bien visible.

Les différentes zones 1, 21, 22, 3 peuvent constituer une unique coque rigide, moulée d'un seul tenant. Elles peuvent être formées de deux coques rigides assemblées et reliées par l'arrière (côté talon donc). L'épaisseur de la coque est de l'ordre de trois millimètres, par exemple plus ou moins deux millimètres. Elle peut être constituée de polyéthylène ou de toute matière plastique moulable et rigide.

Une autre caractéristique intéressante de l'invention réside dans le fait que l'orthèse peut se porter tel quel, sans chaussure, tout en restant totalement efficace. Ceci se révèle très intéressant car simplifie la rééducation.

## Revendications

1. Orthèse talo-tarsienne anatomique destinée à immobiliser l'articulation de la cheville, comprenant plusieurs zones rigides (1, 21, 22, 3) intérieurement recouvertes d'une gaine de contact avec au moins la cheville, au moins un bandeau de liaison (4, 5, 6) destiné à fixer l'orthèse de façon amovible et réglable sur la cheville, lesdites zones rigides (1, 21, 22, 3) étant destinées à immobiliser le calcanéum et le cuboïde et comprenant deux zones de soutien rétro-malléolaire (21, 22) qui permettent le dégagement des deux malléoles,
ainsi qu' une première zone rigide (1) de soutien du soléaire destinée à entourer l'arrière inférieur de la jambe tout en ménageant une ouverture (10) au niveau du tendon d'Achille , cette orthèse étant **caractérisée** ce que ladite ouverture (10) présente une forme en V s'ouvrant depuis l'arrière de la jambe vers le talon, de manière que le talon soit dégagé, non recouvert par l'orthèse.

2. Orthèse selon la revendication 1 **caractérisée en ce qu'**elle comprend une troisième zone rigide (3), de soutien du médio-pied, recouvrant partiellement la surface inférieure du pied, depuis le talon jusqu'au cuboïde.

3. Orthèse selon l'une quelconque des revendications précédentes **caractérisée en ce que** ledit au moins un bandeau de liaison (5, 6) est pourvu de surfaces auto-agrippantes et est prévu pour entourer circonférentiellement la jambe.

4. Orthèse selon l'une quelconque des revendications précédentes **caractérisée en ce que** ledit au moins un bandeau de liaison (4) est pourvu de surfaces auto-agrippantes et est prévu pour entourer la face dorsale du pied.

5. Orthèse selon l'une quelconque des revendications précédentes **caractérisée en ce que** ladite gaine de contact recouvre toute la surface intérieure de l'orthèse.

6. Orthèse selon l'une quelconque des revendications précédentes **caractérisée en ce que** ladite gaine présente une épaisseur comprise entre environ 2 millimètres et environ 6 millimètres.

7. Orthèse selon l'une quelconque des revendications précédentes **caractérisée en ce que** lesdites zones rigides sont constituées en PE ou en une autre matière plastique moulable et rigide.

8. Orthèse selon l'une quelconque des revendications précédentes **caractérisée en ce que** lesdites zones rigides sont monobloc.

## Patentansprüche

1. Anatomische talotarsale Orthese, die dazu bestimmt ist, die Artikulation des Sprunggelenks zu blockieren, mehrere starre Zonen (1, 21, 22, 3) umfassend, die innen mit einer Manschette zum Kontakt mit mindestens dem Sprunggelenk überdeckt sind, mindestens ein Verbindungsband (4, 5, 6), das dazu bestimmt ist, die Orthese abnehmbar und einstellbar an dem Sprunggelenk zu befestigen, wobei die starren Zonen (1, 21, 22, 3) dazu bestimmt sind, das Fersenbein und das Würfelbein zu blockieren und zwei retromalleoläre Stützzonen (21, 22) umfassen, die das Freigeben der beiden Knöchel ermöglichen, sowie eine erste starre Zone (1) zum Stützen des Soleus, die dazu bestimmt ist, das Bein unten an der Rückseite zu umgeben und dabei eine Öffnung (10) im Bereich der Achillessehne freizulassen, wobei diese Orthese **dadurch gekennzeichnet ist, dass** die Öffnung (10) eine V-Form aufweist, die sich von der Rückseite des Beines zur Ferse hin öffnet, damit die Ferse, nicht von der Orthese bedeckt, freigegeben ist.

2. Orthese nach Anspruch 1, **dadurch gekennzeichnet, dass** sie eine dritte starre Zone (3) zum Stützen des Mittelfußes umfasst, die die untere Fläche des Fußes, von der Ferse bis zum Würfelbein teilweise abdeckt.

3. Orthese nach einem der vorstehenden Ansprüche, **dadurch gekennzeichnet, dass** das mindestens eine Verbindungsband (5, 6) mit selbsthaftenden Klettflächen versehen ist, und vorgesehen ist, um das Bein umfänglich zu umgeben.

4. Orthese nach einem der vorstehenden Ansprüche, **dadurch gekennzeichnet, dass** das mindestens eine Verbindungsband (4) mit selbsthaftenden Klettflächen versehen ist, und vorgesehen ist, um die dorsale Seite des Fußes zu umgeben.

5. Orthese nach einem der vorstehenden Ansprüche, **dadurch gekennzeichnet, dass** die Manschette zum Kontakt die gesamte Innenfläche der Orthese abdeckt.

6. Orthese nach einem der vorstehenden Ansprüche, **dadurch gekennzeichnet, dass** die Manschette eine Dicke aufweist, die zwischen etwa 2 Millimetern und etwa 6 Millimetern enthalten ist.

7. Orthese nach einem der vorstehenden Ansprüche, **dadurch gekennzeichnet, dass** die starren Zonen aus PE oder aus einem anderen formbaren und starren Kunststoffmaterial gebildet sind.

8. Orthese nach einem der vorstehenden Ansprüche, **dadurch gekennzeichnet, dass** die starren Zonen aus einem Stück sind.

## Claims

1. An anatomical talotarsal orthosis for immobilising the ankle joint, comprising several rigid areas (1, 21, 22, 3) lined with a sheath that comes into contact with at least the ankle, at least one connecting band (4, 5, 6) for attaching the orthosis in a removable and adjustable manner on the ankle, said rigid areas (1, 21, 22, 3) being for immobilising the calcaneus and the cuboid bone and comprising two retromalleolar support areas (21, 22) which allow the two malleoli to be bare, as well as a first rigid area (1) for supporting the soleus for surrounding the lower rear of the leg while arranging an aperture (10) at the Achilles tendon, this orthosis being **characterised in that** said aperture (10) has a V-shape opening from the rear of the leg towards the heel, so that the heel is bare, not covered by the orthosis.

2. The orthosis according to claim 1 **characterised in that** it comprises a third rigid area (3), for supporting the midfoot, partially covering the lower surface of the foot, from the heel to the cuboid bone.

3. The orthosis according to any one of the preceding claims **characterised in that** said at least one connecting band (5, 6) is provided with hook-and-loop surfaces and is intended to circumferentially surround the leg.

4. The orthosis according to any one of the preceding claims **characterised in that** said at least one connecting band (4) is provided with hook-and-loop surfaces and is intended to surround the dorsal face of the foot.

5. The orthosis according to any one of the preceding claims **characterised in that** said contact sheath covers the entire interior surface of the orthosis.

6. The orthosis according to any one of the preceding claims, **characterised in that** said sheath has a thickness comprised between about 2 millimetres and about 6 millimetres.

7. The orthosis according to any one of the preceding claims, **characterised in that** said rigid areas are made of PE or another mouldable and rigid plastic material.

8. The orthosis according to any one of the preceding claims **characterised in that** said rigid areas are formed in one piece.
